(19) Europäisches Patentamt

European Patent Office

Office européen des brevets

(11) **EP 1 351 605 B1**

(12) **EUROPEAN PATENT SPECIFICATION**

(45) Date of publication and mention
of the grant of the patent:
**27.04.2005 Bulletin 2005/17**

(51) Int Cl.⁷: **A61B 5/16**, A61B 5/02,
A61B 5/00

(21) Application number: **02716099.3**

(22) Date of filing: **04.01.2002**

(86) International application number:
**PCT/FI2002/000007**

(87) International publication number:
**WO 2002/054954 (18.07.2002 Gazette 2002/29)**

(54) **DEVICE FOR DIAGNOSING AND MONITORING OF VEGETATIVE NERVOUS SYSTEM STATE AND FOR USING IN TREATING OF DISEASES AND STATES FEATURED BY HORMONE DISORDER**

GERÄT ZUR DIAGNOSE UND ÜBERWACHUNG DES ZUSTANDS DES VEGETATIVEN NERVENSYSTEMS SOWIE FÜR DIE BEHANDLUNG VON KRANKHEITEN UND LEIDEN AUFGRUND VON HORMONSTÖRUNGEN

DISPOSITIF POUR LE DIAGNOSTIC ET LE CONTROLE DE L'ETAT DU SYSTEME NERVEUX VEGETATIF ET POUR L'UTILISATION DANS LE TRAITEMENT DE MALADIES ET DE PATHOLOGIES CARACTERISEES PAR UN DESORDRE HORMONAL

(84) Designated Contracting States:
**AT BE CH CY DE DK ES FI FR GB GR IE IT LI LU MC NL PT SE TR**

(30) Priority: **09.01.2001 RU 2001100627**

(43) Date of publication of application:
**15.10.2003 Bulletin 2003/42**

(73) Proprietor: **Pulsegate Oy**
**00420 Helsinki (FI)**

(72) Inventors:
• **NAUMOV, Valery, A.**
**St.Petersburg, 193024 (RU)**
• **ILINE, Iouri, Z.**
**St.Petersburg, 194017 (RU)**
• **VIRTANEN, Tauno, O.**
**FIN-00210 Helsinki (FI)**

(74) Representative: **LEITZINGER OY**
**Tammasaarenkatu 1**
**00180 Helsinki (FI)**

(56) References cited:
**EP-A1- 0 885 592**          **EP-A2- 1 059 064**
**DE-U1- 20 017 961**       **SU-A1- 1 364 290**
**US-A- 5 766 130**

• **DATABASE WPI Week 200066, Derwent Publications Ltd., London, GB; AN 2000-678073, XP002966273 & RU 2 151 545 C1 (MAKAROV L.M.) 27 June 2000**
• **DATABASE WPI Week 199642, Derwent Publications Ltd., London, GB; AN 1996-423399, XP002966274 & RU 2 051 617 C1 (ZHULEV S.N.) 10 January 1996**
• **DATABASE WPI Week 199845, Derwent Publications Ltd., London, GB; AN 1998-529626, XP002966275 & RU 2 107 454 C1 (SAMARA ARCHITECTURE BUILDING ACAD) 27 March 1998**

## Description

## Technical Field of the Invention

In accordance with the present invention, a device is provided for diagnosing, monitoring and for using in treating diseases and states featured by hormone disorder, and more specifically a device is provided for diagnosing of patients as having Vegetative nervous system (VNS) state corresponding to drug and toxic substances abuse, alcoholism, drugs and other substances intoxication and hormone disorder due to disease or natural processes in human organism and as having VNS state corresponding to absence of these diseases and states.

## Background of the Invention

Some diseases have a feature of strained Vegetative nervous system (VNS) state (by hormone disorder). Diagnosing of VNS strained state can serve as a first step in diagnosing of diseases and states featured by hormone disorder. This first step at least separates patients into two groups: a group consisting of those with unstrained VNS and a group consisting of those with strained VNS, the latter being an indicator of corresponding diseases and states probable presence and of a necessity to do further more specific tests to diagnose these diseases and states. Quantitative estimation of VNS strain can serve for monitoring of patient's VNS state, this monitoring can be used in its turn in treating of patients with diseases and states featured by hormone disorder to estimate efficiency of treating methods and medicines used if this efficiency is indicated by increasing or decreasing of VNS strain.

A special interest is that for a method and device requiring several minutes to detect drug and toxic substances abuse, alcoholism, drugs and other substances intoxication at workplace or at pre-employment search in case the said diseases and states are intolerable. Those skilled in the art know various drug and their traces tests from hair, saliva and urine analyses and corresponding kits to other complicated, time consuming and expensive methods. Devices for these analyses are also known. As a rule, simplest and thus cheap methods and devices are selective and not very reliable, thus they require series tests for various substances and sometimes other more reliable confirming methods and devices using. Thus, complete tests cost is increased. The said is especially noticeable in case of mass tests. Cost of sophisticated methods and devices is a question according to definition.

The said above can be easily extended for other diseases and states.

For those skilled in the art, various methods and devices for VNS state estimation are known. Accurate and reliable results are obtained by biochemical analysis of catecholamides in a body liquid phase (A.G. Resnikov, "The methods of hormones determination", Naukova Dumka, Kiew, 1980). Being a powerful diagnosing means, this method is complex, much labor and time consuming and expensive. The same can be said about a device used in this method.

Nowadays, non-invasive methods and devices are preferable, sometimes being the only ones tolerating the conditions and requirements of screening.

Document SU-A-1 364 290 discloses a method of determining the condition of the human vegetative nervous system involving recording and evaluating the cardiac time intervals between the R-R waves and determining the index of tension before and after load.

The present invention was stimulated by a call for a non-invasive device featured by low cost, several minutes required for test and at least giving a possibility to take out of further more sophisticated methods tests those who have VNS state with no correspondence to diseases and states featured by strained VNS (hormone disorder).

Therefore one of the present invention objects is to provide a device for VNS state estimation featured by quantitative results data, the latter being in absolute values and with probability ascribed.

## Summary of the Invention

In accordance with the present invention, a non-invasive device for diagnosing, monitoring and for using in treating of diseases and states, in particular of drugs and toxic substances abuse, alcoholism, drugs and other substances intoxication and hormone disorder due to a disease or natural processes in human organism, said device comprising (1) a non-invasive means measuring vascular (cardiovascular) parameters characterizing VNS state, (2) analysis means for calculating of VNSSF values from the measured vascular (cardiovascular) parameters values, (3) analysis means for calculating (determination) of VNSSF and/or the said parameters values bands corresponding to different measurements series with inherent cutoff values limiting the bands, (4) analysis means for comparing of the patient's VNSSF and/or the said parameters values against that within the bands in (3) above, against limiting (cutoff) VNSSF and/or the said parameters values of these bands and against the patient's VNSSF and/or the said parameters values determined earlier, and (5) analysis means for diagnosing the patient as corresponding at least potentially to at least one of the said measurements series or as having increasing or decreasing VNS strain in time.

A method is described for diagnosing of drugs and toxic substances addiction, alcoholism, drugs and other substances intoxication and hormone disorder due to a disease or natural processes in human organism or to at least for separation of persons into two groups - those with strained VNS state and unstrained VNS state corresponding to drugs and toxic substances addiction, alcoholism, drugs and other substances intoxication and hormone disorder due to a disease or natural processes in human organism and to absence of the said diseases and states, respectively.

The method can be used to diagnose with some universal cutoff value of VNSSF and/or of other parameters characterizing VNS state, but such an approach can be accompanied by increasing of diagnosing mistake probability or by false identification a patient as belonging to a group to which he does not belong (depending upon a particular cutoff value chosen). The said is due to the following fact: Statistical data for RG1 show that VNSSF average and maximal values can differ for populations of different countries and even of different climatic zones regions of large territories countries due to genetic features and different prevalent food, living standards and health care. Thus, to increase the accuracy of the method, statistical data for RG1 is desirable to be gathered for the country (region) where the method is planned to be used. The said is not valid, however, for drug abuse and drug users groups, because it was found that drugs impact upon VNS (causing hormones disorder) is rather heavy to push VNS to evidently strained state never mind hypothetical unstrained state for drug abuse and drug users.

Further object of the present invention is a device with quantitative estimation of VNS state, giving a possibility to determine a probability for a patient with a definite VNS state quantitative estimate to correspond to one of the above groups.

The probability thus determined can be used to meet a device according to the present invention to users requirements to the results of diagnosing or separation of patients into two groups said above. Indeed, shifting of a determined cutoff value in direction to the band for RG1 will lead to decreasing of strained VNS state to be determined as unstrained probability and to increasing of false diagnosing of strained VNS state probability, and visa versa. Consider as an example a practical use of the said separation into drug abuse and drug-free persons screening. Normally, the aim is to determine during several seconds or minutes persons with strained VNS state; false unstrained VNS diagnosing probability is to be near zero. These persons are said to be drug-free and are not tested further by other screening methods or devices or by other confirming methods or devices being exact diagnosing means but possessing no advantages set inherent to a method and a drvice according to the present invention (fast&cheap and near-zero false negative results probability being a feature of a special interest for tests at workplaces). Using of a method and a device according to the invention saves time and money. The more is the said cutoff value shift, the more the requirement of low false negative result probability is satisfied. From the other hand, the more is the said cutoff value shift, the less share of tested persons is said to be drug-free, thus the less is overall money and time saving (but the less is a probability to miss a drug abuse). And visa versa. A compromise choice is after the method and a device according to the present invention users specific demand.

Being used prior to test methods/devices possessing unsatisfactory false negative or false positive test results probability, a device according to the present invention made to have near-zero corresponding false results probability improves overall corresponding false results probability. Indeed, after separation into two above said groups, only a part of all tested persons is then tested with unsatisfactory false results probability.

Further object of the present invention is a device to be used in treatment of diseases and states featured by strained VNS for treatment methods and medicines used efficiency estimation in case a progress or regress can be estimated by a response of VNS state to treatment methods and medicines.

Further object of the present invention is a device, which uses, along with other vascular (cardiovascular) parameters characterizing VNS state, an invented VNSSF, found to be a reliable quantitative parameter of VNS strain (hormone disorder). VNSSF can be determined using data received from different principles sensors measuring vascular (cardiovascular) parameters, including blood volume (BV) and pressure (BP), blood volume and pressure variation velocity (BV/dt & BP/dt) and blood volume and pressure variation acceleration (BV/dt2 & BP/dt2). A particular parameter choice made to achieve maximal sensitivity depends upon the sensors in non-invasive means measuring vascular (cardiovascular) parameters) to be used with a particular tests conditions and other factors in mind. It is evident also for those skilled in the art that particular practical sensors characteristics (frequency passband, etc.) can result in different parameters values determined directly from, for example, BP and BP/dt sensors and from one of the said sensors with following differentiation or integration. Thus, to improve accuracy of the method, VNSSF is better to be determined for a particular sensor type and a particular signal from the sensor processing.

Further object of the present invention is a device requiring no separate means for analog-to digital transformation and for dc power supply to amplifying means. These functions are after computer.

Further object of the present invention is a device excluding human element mistakes due a possibility of computerized data treatment and diagnosing.

These and other features will become apparent to those skilled in the art upon a review of the detailed description of a preferred embodiment of the present invention presented below, in conjunction with the drawings presented herewith.

**Brief description of the drawings**

Fig. 1    is BP/dt vs. time response typical for persons with unstrained VNS state.

Fig. 2    is BP/dt vs. time response typical for persons with strained VNS state.

Fig. 3    is VNSSF value distribution for drug-free and drugs abuse groups.

Fig. 4    is VNSSF value probabilities to correspond to drug-free and to drugs abuse.

Fig. 5    is VNSSF integral probabilities for drug-free and for drugs abuse.

Fig. 6    is a device according to the present invention.

Fig. 7    is a device according to preferred embodiment of the present invention.

## Detailed description of the preferred embodiment

Consider working example and experimental results. This example concerns drug screening tests but the device according to the present invention using can be readily expanded on diagnosing of other diseases and states featured by strained VNS state (hormone disorder).

Many years practice proved that in case of hormone disorder (strained VNS state), a correlation of left ventricle and aorta contribution to blood flow varies in favor of the latter when compared with that for RG1. It could be noticed, in particular, by analyzing BV vs time response measured by photoplethysmographic sensor affixed to a finger tip or other part of human body. However, this effect of difference between vascular (cardiovascular) parameters responses for strained and unstrained VNS was noticed to be more pronounced in case of using differential pressure measuring sensors with output signal corresponding to BP/dt.

Preferred embodiment of the present invention is described for a case of practical drugs abuse screening (or prescreening if some other screening tests are made further).

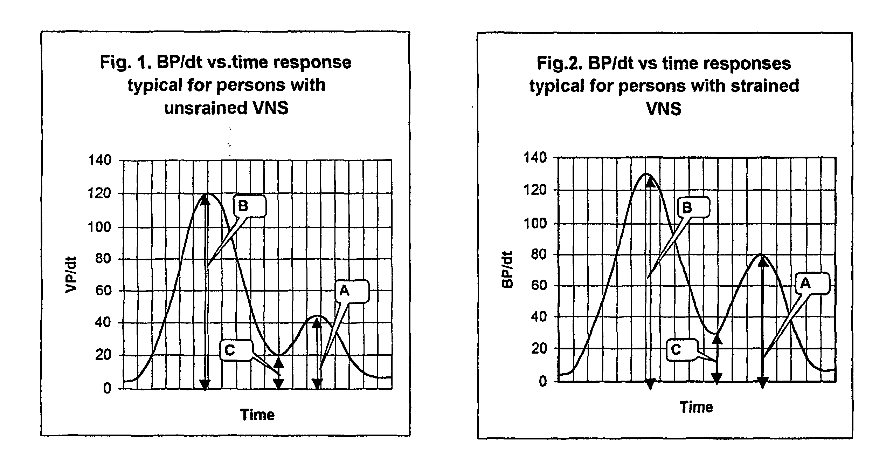

In Figs. 1 and 2, typical BP/dt (with a constant component added) vs. time responses for a typical unstrained VNS and strained VNS are shown, respectively. BP/dt values were measured by a non-invasive means directly (not via differentiation of measured BP, for example). Fig 2 corresponds to drugs abuse, in particular. Non-invasive BaTi differential pressure sensor was used. These Figs are that in the screen of a computer. This computer was used was used also as a source of dc voltage required for an amplifier built into the sensor. Depending upon power requirements, Com or USB port can be used. Analog-to-digit transformation of a signal from the sensor was produced in a computer with no separate or built into the sensor corresponding unit. No separate dc voltage source and analog-to-digit transforming unit reduces a cost of measurement device. Detailed description of a device used in present preferred embodiment is cited below.

The said in the above paragraph is true for all the measurements considered below.

One of the features of the present invention is VNSSF calculation and using in diagnosing. This parameter was found to be an easily detectable and a reliable parameter characterizing VNS state. In preferred embodiment of the present invention, calculations of VNSSF values and all the operations with them and/or other said parameters are made by a computer.

VNSSF according to the present invention is thus equal to:

$$I = (A-C)/(B-C),$$

where

I    is VNSSF,
A    is the BP/dt max. value corresponding to aorta push (contraction),
B    is the BP/dt max. value corresponding to left heart ventricle push (contraction),
C    is the BP/dt max. value corresponding to closing of aorta valve.

In Figs. 1 and 2, A, B and C for the above expression are indicated giving I values 0,24 and 0,56, respectively.

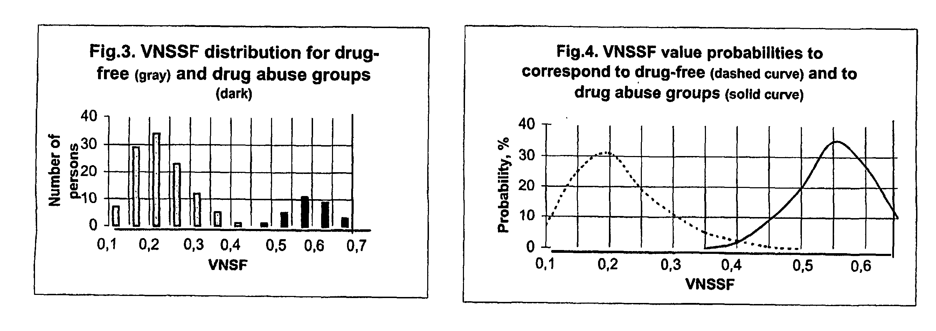

VNS state information according to the present invention was gathered from RG1 and drugs abuse (heroin) groups (RG2) in St.-Petersburg, Russia. VNSSF was determined from the gathered VNS state information. The data for VNSSF thus obtained are shown in Fig.3. These data were treated to determine distribution law(s) of VNSSF for the said groups distribution. For those skilled in the art, it is evident, that with a particular distribution law (Gaussian random values distribution, for example) known, all probability estimates could be calculated or obtained directly from reference books. Thus, distribution law(s) determination gives a possibility to determine probability of a person's particular VNSSF value to fall within corresponding band of VNSSF, that is to determine quantitatively probability of a person's particular VNSSF value to correspond to strained or unstrained VNS state (drug abuse or drug-free groups, respectively, in the considered case). Determination of a distribution law and quantitative probability calculations were made by a computer.

In Fig.4, illustrative responses for probabilities of a particular VNSSF value to correspond to that for drug-free and to that for drug abuse are shown. These responses are plotted using data of Fig.3 for determination of VNSSF values distribution law and mathematical estimate of dissipation for the distribution law.

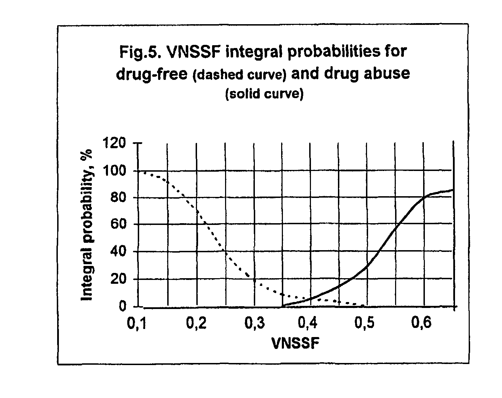

Data of Fig.4 can be used for plotting the responses of Fig.5, the latter show integrated probabilities values. For drug-free, integration is made in direction from infinite VNNSF value to 0. For drug abuse, integration is made in direction from 0 to infinite VNSSF value.

What particular probability value is used further for determination of a cutoff VNSSF value, depends upon a method/device according to the present invention users' requirements. In case of practical separation of screened persons into two groups with drug-free taken out from further tests by other methods, the probability of false negative result (drug abuse is said to be drug-free) is of a primary importance. This probability value is usually to be as low as it is possible.

Fig.5 gives a possibility to choose VNSSF cutoff level (separating VNSSFs to those for drug-free and for drugs abuse) to meet a device according to the present invention user's requirements expressed quantitatively.

Analysis of Fig.5 shows that, for example,

- with cutoff VNSSF value equal to 0,30, probability to false negative result is 0% with a reserve (0,35 value corresponds to 0%), and probability of false positive result (drug-free is said to be t drug abuse) equals 20%.
- with cutoff VNSSF value equal to 0,35, a probability to false negative result is near 0%, and probability of false positive result equals 9%.
- with cutoff VNSSF value equal to 0,40, a probability to false negative result equals 5%, and probability of false positive result equals 5% also.

Particular requirements of a user of a device according to the present invention can differ substantially depending upon specific screening (prescreening) requirements and a particular method/device to be further used. In case some other cheap method/device with very small probability of false positive result obtaining planned to be used after a device according to the present invention, choosing of VNSSF cutoff level equal 0,3 or even lower is substantiated. Indeed, it is not a problem to test further 20% again to confirm or to correct preceding test results and to obtain very near to zero false positive and false negative results, that is accuracy and specificity of test procedure (two said tests) at an acceptable level. In case some sophisticated expensive method/device is further used, each extra test can increase overall test procedure cost noticeably, and choosing of VNSSF cutoff level equal to 0,35 looks to be more reasonable (corresponding to 9% of persons to be further tested). In any way, false positive results probability seems to be predetermined with near zero value, thus maximal VNSSF cutoff level is 0,35.

As it can be seen from the above quantitative estimation, from 80% to 91% of tested persons could be taken off from further tests. Certainly, these figures can differ in practical cases for many reasons, including a share of drug abuses in screened volume, but substantial reducing of test procedure/device cost and improving of further used method/device characteristics if they are not satisfactory (false negative and/or false positive results probability) are evident.

It should be noted also that using in a device according to the present invention of other vascular (cardiovascular) parameters along with VNSSF, may improve quantitative estimates in a preferred embodiment. For example, heart rate measurements can take out of further tests those with high VNSSF but with bradycardia, because drug abuse is featured (at least heroin abuse) by expressed tachycardia.

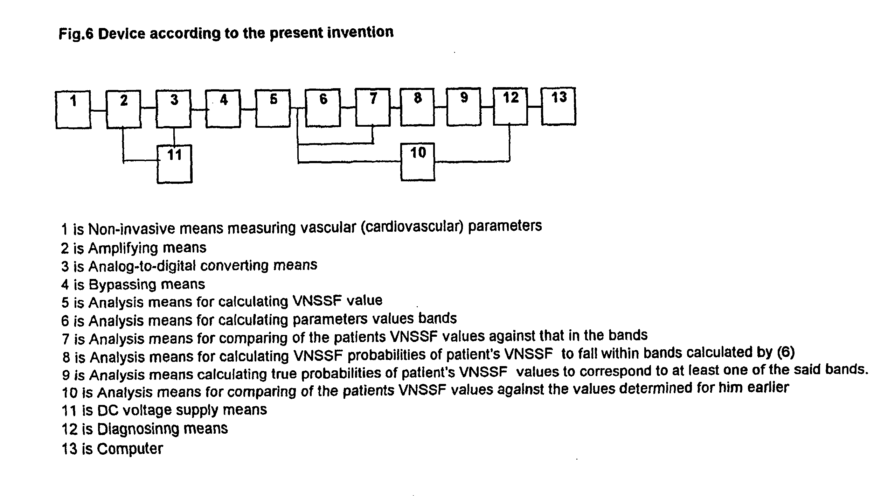

In Fig.6, a flow chart of a device according to the present invention is shown. In fig. 6 reference numeral 1 is a non-invasive means measuring vascular (cardiovascular) parameters, reference numeral 2 is an amplifying means, reference numeral 3 is an analog-to-digital converting means, reference numeral 4 is a bypassing means, reference numeral 5 is an analysis means for calculating VNSSF value, 6 is an analysis means for calculating parameters values bands, reference numeral 7 is an analysis means for comparing of the patients VNSSF values against that in the bands, reference numeral 8 is an analysis means for calculating VNSSF probabilities of patient's VNSSF to fall within bands calculated by (6), reference numeral 9 is an analysis means calculating true probabilities of patient's VNSSF values to correspond to at least one of the said bands, reference numeral10 is an analysis means for comparing of the patients VNSSF values against the values determined for him earlier, reference numeral 11 is DC voltage supply means, reference numeral 12 is diagnosing means and reference numeral 13 is a computer.

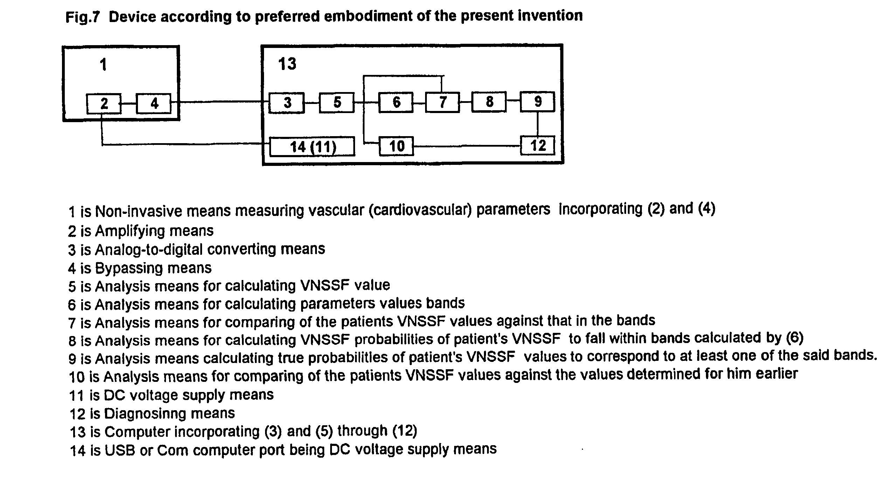

In Fig.7, a flow chart of a device according to a preferred embodiment of the present invention is shown. In this device, a non-invasive means measuring vascular (cardiovascular) parameters incorporates amplifying and bypassing means 2 and 4; computer USB or Com port 14 serves as DC voltage supply means and this computer incorporates all the rest means in Fig.6 using standard and special programs.

According to the present invention it is possible to connect a non-invasive means directly or via other means to an oscilloscope and/or to a computer.

The device of a preferred embodiment of the present invention comprises a converting means for analog-to-digital conversion of the signal from said non-invasive means. Said converting means may be incorporated with said non-invasive means. Preferably said converting means is a computer.

The device of a preferred embodiment of the present invention comprises also an amplifying means, said amplifying means being preferably incorporated with the said non-invasive means.

The device of a preferred embodiment of the present invention comprises also a bypassing means for blocking constant component of said non-invasive means signal from a computer.

The device of a preferred embodiment of the present invention comprises further a power supply means supplying dc voltage for said transforming means and/or for the said amplifying means, said power supply means being an oscilloscope or a computer.

Means for calculating VNSSF value is preferably incorporated with the said non-invasive means. Said calculating means is a computer and said analysis means for comparing of the patient's VNSSF and/or the said parameters values is also a computer.

Although the invention has been described in detail with reference to the illustrated preferred embodiments, variations and modifications exist within the scope of the invention as described and as defined in the following claims.

**Claims**

1. A non-invasive device for diagnosing, monitoring and for using in treating of diseases and states, in particular of drugs and toxic substances abuse, alcoholism, drugs and other substances intoxication and hormone disorder due to a disease or natural processes in human organism, said device comprising (1) a non-invasive means measuring vascular parameters **characterizing** VNS state, (2) analysis means for calculating of VNSSF values from the measured vascular parameters values, **characterized in that** the device comprises (3) analysis means for calculating of VNSSF and/or the said parameters values bands corresponding to different measurements series with inherent cutoff values limiting the bands, (4) analysis means for comparing of the patient's VNSSF and/or the said parameters values against that within the bands in (3) above, against limiting cutoff VNSSF and/or the said parameters values of these bands and against the patient's VNSSF and/or the said parameters values determined earlier, and (5) analysis means for diagnosing the patient as corresponding at least potentially to at least one of the said measurements series or as having increasing or decreasing VNS strain in time.

2. The device of claim 1 further including analysis means calculating probabilities of patient's VNSSF and/or the said parameters values to fall within the said bands.

3. The device of claim 1 further including analysis means calculating true probabilities of patient's VNSSF and/or the said parameters values to correspond to at least one of the said bands.

4. The device of claim 1 wherein the said non-invasive means is non-invasive means measuring at least one of the following group of vascular /cardiovascular parameters: total vascular conductance, vascular elasticity, large artery elasticity index, small artery elasticity index, systemic vascular conductance pulse rate, or a combination thereof.

5. The device of claim 1 wherein the said non-invasive means is a non-invasive means measuring BV peaks and valleys within at least one full heart stroke period, at least peaks of BV due to left heart ventricle and aorta and valley corresponding to aorta valve closing and/or BV waveforms.

6. The device of claim 1 wherein the said non-invasive means is a non-invasive means measuring BV/dt peaks and valleys within at least one full heart stroke period, at least peaks of BV/dt due to left heart ventricle and aorta and valley corresponding to aorta valve closing and/or BV/dt waveforms.

7. The device of claim 1 wherein the said non-invasive means is a non-invasive means measuring BP peaks and valleys within at least one full heart stroke period, at least peaks of BP due to left heart ventricle and aorta and valley corresponding to aorta valve closing and/or BP waveforms.

8. The device of claim 1 wherein the said non-invasive means is a non-invasive means measuring BP/dt peaks and valleys within at least one full heart stroke period, at least peaks of BP/dt due to left heart ventricle and aorta and valley corresponding to aorta valve closing and/or BP/dt waveforms.

9. The device of claim 1 wherein the said non-invasive means is a non-invasive means measuring BV/dt2 peaks and valleys within at least one full heart stroke period, at least peaks of BV/dt2 due to left heart ventricle and aorta and valley corresponding to aorta valve closing and/or BV/dt2 waveforms.

10. The device of claim 1 wherein the said non-invasive means is a non-invasive means measuring BP/dt2 peaks and valleys within at least one full heart stroke period, at least peaks of BP/dt2 due to left heart ventricle and aorta and valley corresponding to aorta valve closing and/or BP/dt2 waveforms.

11. The device of claims 5 through 10 wherein the said non-invasive means is selected from the following group: a photoplethysmograph probe, a physical pressure measuring, a rheograph probe, an ultrasonic probe, and an X-ray probe.

12. The device of claims 5 and 11 further including calculating means transforming BV vs time responses into BV/dt vs time responses by differentiation.

**13.** The device of claims 6 and 11 further including calculating means transforming BV/dt vs time responses into BV vs time responses by integration.

**14.** The device of claims 5 and 11 further including calculating means transforming BV vs time responses into BV/dt2 vs time responses by double differentiation.

**15.** The device of claims 6 and 11 further including calculating means transforming BV/dt vs time responses into BV/dt2 vs time responses by differentiation.

**16.** The device of claims 9 and 11 further including calculating means transforming BV/d2 vs time responses into BV/dt vs time responses by integration.

**17.** The device of claims 9 and 11 further including calculating means transforming BV/d2 vs time responses into BV vs time responses by double integration.

**18.** The device of claims 7 and 11 further including calculating means transforming BP vs time responses into BP/dt vs time responses by differentiation.

**19.** The device of claims 8 and 11 further including calculating means transforming BP/dt vs time responses into BP vs time responses by integration.

**20.** The device of claims 7 and 11 further including calculating means transforming BP vs time responses into BP/dt2 vs time responses by double differentiation.

**21.** The device of claims 8 and 11 further including calculating means transforming BP/dt vs time responses into BP/dt2 vs time responses by differentiation.

**22.** The device of claims 10 and 11 further including calculating means transforming BP/dt2 vs time responses into BP/dt vs time responses by integration.

**23.** The device of claims 10 and 11 further including calculating means transforming BP/dt2 vs time responses into BP vs time responses by double integration.

**24.** The device of claims 5, 13 and 17 wherein the said analysis means for calculating of the VNSSF value from the measured vascular parameters values calculates this value according to the following expression:

$$I = (A-C)/(B-C),$$

where

I    is VNSSF,
A   is the BV corresponding to aorta push,
B   is the BV corresponding to left heart ventricle push,
C   is the BV corresponding to closing of aorta valve.

**25.** The device of claim 24 wherein A and B are the maximal values and C is the minimal value of corresponding BV.

**26.** The device of claims 7, 19 and 23 wherein the said analysis means for calculating of the VNSSF value from the measured vascular (cardiovascular) parameters values calculates this value according to the following expression:

$$I = (A-C)/(B-C),$$

where

I    is VNSSF,
A   is the BP corresponding to aorta push,
B   is the BP corresponding to left heart ventricle push,
C   is the BP corresponding to closing of aorta valve.

**27.** The device of claim 26 wherein A and B are the maximal values and C is the minimal value of corresponding BP.

**28.** The device of claims 6, 12 and 16 wherein the said analysis means for calculating of the VNSSF value from the measured vascular parameters values calculates this value according to the following expression:

$$I = (A-C)/(B-C),$$

where

I    is VNSSF,
A   is the BV/dt corresponding to aorta push,
B   is the BV/dt corresponding to left heart ventricle push,
C   is the BV/dt corresponding to closing of aorta valve.

**29.** The device of claim 28 wherein A and B are the maximal values and C is the minimal value of corresponding BV/dt.

**30.** The device of claims 8, 18 and 22 wherein the said analysis means for calculating of the VNSSF value from the measured vascular parameters values calculates this value according to the following expression:

$$I = (A-C)/(B-C),$$

where

I   is VNSSF,
A   is the BP/dt corresponding to aorta push,
B   is the BP/dt corresponding to left heart ventricle push,
C   is the BP/dt corresponding to closing of aorta valve.

31. The device of claim 30 wherein A and B are the maximal values and C is the minimal value of corresponding BP/dt.

32. The device of claims 9, 14 and 15 wherein the said analysis means for calculating of the VNSSF value from the measured vascular parameters values calculates this value according to the following expression:

$$I = (A-C)/(B-C),$$

where

I   is VNSSF,
A   is the BV/dt2 corresponding to aorta push,
B   is the BV/dt2 corresponding to left heart ventricle push,
C   is the BV/dt2 corresponding to closing of aorta valve.

33. The device of claim 32 wherein A and B are the maximal values and C is the minimal value of corresponding BV/dt2.

34. The device of claims 10, 20 and 21 wherein the said analysis means for calculating of the VNSSF value from the measured vascular parameters values calculates this value according to the following expression:

$$I = (A-C)/(B-C),$$

where

I   is VNSSF,
A   is the BP/dt2 corresponding to aorta push,
B   is the BP/dt2 corresponding to left heart ventricle push,

C   is the BP/dt2 corresponding to closing of aorta valve.

35. The device of claim 34 wherein A and B are the maximal values and C is the minimal value of corresponding BP/dt2.

36. The device of claim 1 wherein the said analysis means for diagnosing the patient, diagnosis VNNSF exceeding 0,3, being measured at maximal A and B values, as corresponding to strained VNS state due to drugs and toxic substances abuse, alcoholism, drugs and other substances intoxication and hormone disorder due to a disease or natural processes in human organism.

37. The device of claim 11 wherein said non-invasive means is connected directly or via other means to an oscilloscope and/or directly or via other means to a computer.

38. The device of claim 11 further comprising a converting means for analog-to-digital conversion of the signal from the said non-invasive means.

39. The device of claim 38 wherein the said converting means is incorporated with the said non-invasive means.

40. The device of claim 38 wherein the said converting means is a computer.

41. The device of claim 11 further comprising an amplifying means.

42. The device of claim 41 wherein the said amplifying means is incorporated with the said non-invasive means.

43. The device of claim 11 and 37 further comprising a bypassing means for blocking constant component of the said non-invasive means signal from the said oscilloscope and/or computer.

44. The device of claims 38 through 42 further comprising a power supply means supplying dc voltage for the said converting means and/or for the said amplifying means, said dc voltage supplying means being selected from the following group: an oscilloscope or a computer.

45. The device of claims 12 through 23 wherein the said calculating means is incorporated with the said non-invasive means.

46. The device of claims 12 through 23 wherein the said calculating means is a computer.

**47.** The device of claim 1 wherein a computer is used as said analysis means for calculating of VNSSF and/or the said parameters values bands, and/or for comparing of the patient's VNSSF and/or the said parameters values and/or for diagnosing the patient.

**Patentansprüche**

**1.** Nicht invasive Vorrichtung zur Diagnose, Überwachung und zur Verwendung bei der Behandlung von Krankheiten und Zuständen, insbesondere bei Missbrauch von Drogen und toxischen Substanzen, Alkoholismus, Vergiftung durch Medikamente und andere Substanzen und Hormonstörungen aufgrund einer Krankheit oder natürlicher Vorgänge im menschlichen Organismus, mit (1) einem nicht invasiven Mittel, das Gefässparameter zur Kennzeichnung des VNS-Zustands misst, (2) Analysemittel zur Berechnung von VNS-Stressorenwerten aus den gemessenen Gefäßparameterwerten, **gekennzeichnet dadurch, dass** die Vorrichtung folgendes umfasst: (3) Analysemittel zur Berechnung der VNS-Stressoren und/oder der besagten Parameterwertbänder entsprechend verschiedenen Reihen von Messungen mit inhärenten Cut-off-Werten, die die Bänder begrenzen, (4) Analysemittel zum Vergleichen der VNS-Stressoren des Patienten und/oder der besagten Parameterwerte mit denen in den Bändern in (3) oben, mit Grenz-Cut-off-VNS-Stressorenwerten und/oder den besagten Parameterwerten dieser Bänder und mit den VNS-Stressoren des Patienten und/oder den vorher bestimmten Parameterwerten, und (5) Analysemittel zwecks Diagnose für den Patienten entsprechend zumindest potentiell mindestens einer der besagten Reihen von Messungen oder mit steigendem oder sich verringerndem VNS-Stress über die Zeit.

**2.** Vorrichtung nach Anspruch 1 mit des weiteren Analysemitteln, die Wahrscheinlichkeiten der VNS-Stressoren des Patienten berechnen und/oder die besagten Parameterwerte, die in besagte Bänder fallen.

**3.** Vorrichtung nach Anspruch 1 mit des weiteren Analysemitteln, die wahre Wahrscheinlichkeiten der VNS-Stressoren des Patienten berechnen und/oder die besagten Parameterwerte, die mindestens einem der besagten Bänder entsprechen.

**4.** Vorrichtung nach Anspruch 1, wobei das besagte nicht invasive Mittel ein nicht invasives Mittel ist, das mindestens eine der folgenden Gruppe von vaskularen / kardiovaskularen Parametern misst: gesamter Gefäßleitwert, Gefäßelastizität, Großgefäß-Elastizitätsindex, Kleingefäß-Elastizitätsindex, systemische Gefäßleitwertpulsfrequenz, oder eine Kombination davon.

**5.** Vorrichtung nach Anspruch 1, wobei das besagte nicht invasive Mittel ein nicht invasives Mittel ist, das BV-Spitzen und Täler innerhalb mindestens einer vollen Herzschlagperiode misst, zumindest Spitzen von BV aufgrund linker Herzkammer und Aorta und Tal entsprechend der Aortaklappendosierung und/oder in Wellenformen.

**6.** Vorrichtung nach Anspruch 1 wobei das besagte nicht invasive Mittel ein nicht invasives Mittel ist, das BV/dt Spitzen und Täler innerhalb mindestens einer vollen Herzschlagperiode misst, zumindest Spitzen von /dt aufgrund linker Herzkammer und Aorta und Tal entsprechend der Aortaklappenschließung und/oder BV/dt Wellenformen.

**7.** Vorrichtung nach Anspruch 1, wobei das besagte nicht invasive Mittel ein nicht invasives Mittel ist, das BP-Spitzen und Täler innerhalb mindestens einer vollen Herzschlagperiode misst, zumindest Spitzen von BP aufgrund linker Herzkammer und Aorta und Tal entsprechend der Aortaklappendosierung und/oder BP-Wellenformen.

**8.** Vorrichtung nach Anspruch 1, wobei das besagte nicht invasive Mittel ein nicht invasives Mittel ist, das BP/dt-Spitzen und Täler innerhalb mindestens einer vollen Herzschlagperiode misst, zumindest Spitzen von BP/dt aufgrund linker Herzkammer und Aorta und Tal entsprechend der Aortaklappenschließung und/oder BP/dt-Wellenformen.

**9.** Vorrichtung nach Anspruch 1 wobei das besagte nicht invasive Mittel ein nicht invasives Mittel ist, das BV/dt2-Spitzen und Täler innerhalb mindestens einer vollen Herzschlagperiode misst, zumindest Spitzen von BV/dt2 aufgrund linker Herzkammer und Aorta und Tal entsprechend der Aortaklappenschließung und/oder BV/dt2-Wellenformen.

**10.** Vorrichtung nach Anspruch 1, wobei das besagte nicht invasive Mittel ein nicht invasives Mittel ist, das BP/dt2 Spitzen und Täler innerhalb mindestens einer vollen Herzschlagperiode misst, zumindest Spitzen von BP/dt2 aufgrund linker Herzkammer und Aorta und Tal entsprechend der Aortaklappenschließung und/oder BP/dt2-Wellenformen.

**11.** Die Vorrichtung von Patentansprüchen 5 durch 10, wobei das besagte nicht invasive Mittel aus der folgenden Gruppe ausgewählt ist: eine Photoplethysmographsonde, eine physikalische Druckmessung, eine Rheographsonde, eine Ultraschallsonde und eine Röntgensonde.

**12.** Vorrichtung nach den Ansprüchen 5 und 11 mit des weiteren einem Rechenmittel, das durch Differenzierung BV-vs-Zeitreaktionen in BV/dt-vs-Zeitreaktionen umwandelt.

**13.** Vorrichtung nach den Ansprüchen 6 und 11 mit des weiteren einem Rechenmittel, das BV/dt-vs-Zeitreaktionen in BV-vs-Zeitreaktionen durch Integration umwandelt.

**14.** Vorrichtung nach den Ansprüchen 5 und 11 mit des weiteren einem Rechenmittel, das BV-vs-Zeitreaktionen in BV/dt2-vs-Zeitreaktionen durch doppelte Differenzierung umwandelt.

**15.** Vorrichtung nach den Ansprüchen 6 und 11 mit des weiteren einem Rechenmittel, das BV/dt-vs-Zeitreaktionen in BV/dt2-vs-Zeitreaktionen durch Differenzierung umwandelt.

**16.** Vorrichtung nach den Ansprüchen 9 und 11 mit des weiteren einem Rechenmittel, das BV/d2-vs-Zeitreaktionen in BV/dt-vs-Zeitreaktionen durch Integration umwandelt.

**17.** Vorrichtung nach den Ansprüchen 9 und 11 mit des weiteren einem Rechenmittel das BV/d2-vs-Zeitreaktionen in BV-vs-Zeitreaktionen durch doppelte Integration umwandelt.

**18.** Vorrichtung nach den Ansprüchen 7 und 11 mit des weiteren einem Rechenmittel 15', das BP-vs-Zeitreaktionen in BP/dt-vs-Zeitreaktionen durch Differenzierung umwandelt.

**19.** Vorrichtung nach den Ansprüchen 8 und 11 mit des weiteren einem Rechenmittel, das BP/dt-vs-Zeitreaktionen in BP-vs-Zeitreaktionen durch Integration umwandelt.

**20.** Vorrichtung nach den Ansprüchen 7 und 11 mit des weiteren einem Rechenmittel, das BP-vs-Zeitreaktionen in BP/dt2-vs-Zeitreaktionen durch doppelte Differenzierung umwandelt.

**21.** Vorrichtung nach den Ansprüchen 8 und 11 mit des weiteren einem Rechenmittel, das BP/dt-vs-Zeitreaktionen in BP/dt2-vs-Zeitreaktionen durch Differenzierung umwandelt.

**22.** Vorrichtung nach den Ansprüchen 10 und 11 mit des weiteren einem Rechenmittel, das BP/dt2-vs-Zeitreaktionen in BP/dt-vs-Zeitreaktionen durch Integration umwandelt.

**23.** Vorrichtung nach den Ansprüchen 10 und 11 mit des weiteren einem Rechenmittel, das BP/dt2-vs-Zeitreaktionen in BP-vs-Zeitreaktionen durch doppelte Integration umwandelt.

**24.** Vorrichtung nach den Ansprüchen 5, 13 und 17, wobei das besagte Analysemittel zur Berechnung des VNS-Stressoren-Werts aus den gemessenen Gefäßparameterwerten diesen Wert gemäß dem folgenden Ausdruck berechnet:

$$I = (A-C)/(B-C),$$

wobei I die VNS-Stressoren sind,
A ist der BV-Wert entsprechend dem Aortaschub,
B ist der BV-Wert entsprechend dem linken Herzkammerschub,
C ist der BV-Wert entsprechend der Aortaklappendosierung.

**25.** Vorrichtung nach Anspruch 24, wobei a und b die maximalen Werte sind und C der minimale Wert des entsprechenden BV ist.

**26.** Vorrichtung nach den Ansprüchen 7, 19 und 23, wobei das besagte Analysemittel zur Berechnung des VNS-Stressoren-Werts aus den gemessenen vaskularen (kardiovaskularen) Parameterwerten diesen Wert gemäß dem folgenden Ausdruck errechnet:

$$I = (A-C)/(B-C),$$

wobei I die VNS-Stressoren sind,
A ist der BP-Wert entsprechend dem Aortaschub,
B ist der BP-Wert entsprechend dem linken Herzkammerschub,
C ist der BP-Wert entsprechend der Aortaklappenschließung.

**27.** Vorrichtung nach Anspruch 26 wobei a und b die maximalen Werte sind und C der minimale Wert des entsprechenden BP-Werts ist.

**28.** Vorrichtung nach den Ansprüchen 6, 12 und 16, wobei das besagte Analysemittel 5 zur Berechnung des VNS-Stressoren-Werts aus den gemessenen Gefäßparameterwerten diesen Wert gemäß dem folgenden Ausdruck berechnet:

$$I = (A-C)/(B-C),$$

wobei I die VNS-Stressoren sind,
A ist der BV/dt-Wert entsprechend dem Aortaschub,
B lst der BV/dt-Wert entsprechend dem linken Herzkammerschub,
C ist der BV/dt-Wert entsprechend der Aortaklappendosierung.

**29.** Vorrichtung nach Anspruch 28, wobei A und B die maximalen Werte sind und C der minimale Wert des entsprechenden BV/dt-Werts.

**30.** Vorrichtung nach den Ansprüchen 8, 18 und 22, wobei das besagte Analysemittel zur Berechnung des VNS-Stressoren-Werts aus den gemessenen Gefäßparameterwerte diesen Wert gemäß dem folgenden Ausdruck berechnet:

$$I = (A-C)/(B-C),$$

wobei I die VNS-Stressoren sind,
A ist der BP/dt-Wert entsprechend dem Aortaschub,
B ist der BP/dt-Wert entsprechend dem linken Herzkammerschub,
C ist der BP/dt-Wert entsprechend der Aortaklappendosierung.

**31.** Vorrichtung nach Anspruch 30 wobei A und B die maximalen Werte und C der minimale Wert des entsprechenden BP/dt ist.

**32.** Vorrichtung nach den Ansprüchen 9, 14 und 15, wobei das besagte Analysemittel zur Berechnung des VNS-Stressoren-Werts aus den gemessenen Gefäßparameterwerten diesen Wert gemäß dem folgenden Ausdruck berechnet:

$$I = (A-C)/(B-C),$$

wobei I die VNS-Stressoren sind,
A ist der BV/dt2-Wert entsprechend dem Aortaschub,
B ist der BV/dt2-Wert entsprechend dem linken Herzkammerschub,
C Ist der BV-Wert entsprechend der Aortaklappendosierung.

**33.** Vorrichtung nach Anspruch 32, wobei A und B die maximalen Werte und C der minimale Wert des entsprechenden BV/dt2 ist.

**34.** Vorrichtung nach den Ansprüchen 10, 20 und 21, wobei das besagte Analysemittel zur Berechnung des VNS-Stressoren-Werts aus den gemessenen Gefäßparameterwerten diesen Wert gemäß dem folgenden Ausdruck berechnet:

$$I = (A-C)/(B-C),$$

wobei I die VNS-Stressoren sind,

A ist der BP/dt2-Wert entsprechend dem Aortaschub,
B ist der BP/dt2-Wert entsprechend dem linken Herzkammerschub.
C ist der BP/dt2-Wert entsprechend der Aortaklappenschließung.

**35.** Vorrichtung nach Anspruch 34, wobei A und B die maximalen Werte und C der minimale Wert des entsprechenden BP/dt2 ist.

**36.** Vorrichtung nach Anspruch 1, wobei das besagte Analysemittel zwecks Diagnose für den Patienten VNS-Stressoren über 0,3 diagnostiziert, gemessen bei maximalen A- und B-Werten, entsprechend einem VNS-Stresszustand aufgrund Missbrauchs von Drogen und toxischer Substanzen, Alkoholismus, Vergiftung durch Medikamente und andere Substanzen und Hormonstörung aufgrund einer Krankheit oder natürlicher Vorgänge im menschlichen Organismus.

**37.** Vorrichtung nach Anspruch 11 wobei besagtes nicht invasive Mittel direkt oder durch andere Mittel mit einem Oszilloskop verbunden ist und/oder direkt oder durch andere Mittel mit einem Computer.

**38.** Vorrichtung nach Anspruch 11, des weiteren umfassend ein Umwandlungsmittel für Analog-in-digital-Umwandlung des Signals des nicht invasiven Mittels.

**39.** Vorrichtung nach Anspruch 38, wobei das besagte Umwandlungsmittel in das nicht invasive Mittel integriert ist.

**40.** Vorrichtung nach Anspruch 38, wobei das besagte Umwandlungsmittel ein Computer ist.

**41.** Vorrichtung nach Anspruch 11, des weiteren umfassend ein Verstärkungsmittel.

**42.** Vorrichtung nach Anspruch 41, wobei das besagte Verstärkungsmittel in das nicht invasive Mittel integriert ist.

**43.** Vorrichtung nach Anspruch 11 und 37 des weiteren umfassend ein Umleitmittel zum Blockieren der konstanten Komponente des Signals des nicht invasiven Mittels 25 von dem besagten Oszilloskop und/oder Computer.

**44.** Vorrichtung nach den Ansprüchen 38 durch 42, des weiteren umfassend ein Stromversorgungsmittel das Gleichspannung für das Umwandlungsmittel und/oder für das Verstärkungsmittel liefert, wobei das Gleichspannung liefernde Mittel aus der folgenden Gruppe ausgewählt ist: ein Oszilloskop oder ein Computer.

**45.** Vorrichtung nach den Ansprüchen 12 durch 23, wobei das besagte Rechenmittel in das nicht invasive Mittel integriert ist.

**46.** Vorrichtung nach den Ansprüchen 12 bis 23, wobei das besagte Rechenmittel 5 ein Computer ist.

**47.** Vorrichtung nach Anspruch 1 wobei ein Computer als Analysemittel zur Berechnung der VNS-Stressoren und/oder der besagten Parameterwertbänder benutzt wird, und/oder zum Vergleichen der VNS-Stressoren des Patienten und/oder der Parameterwerte und/oder zwecks Diagnose für den Patienten.

**Revendications**

**1.** Dispositif non invasif pour le diagnostic, le contrôle et l'utilisation dans le traitement de maladies et de pathologies, en particulier d'abus de drogues et de substances toxiques, d'alcoolisme, d'intoxication par médicaments et autres substances, et de désordre hormonal consécutif à une maladie ou à un processus naturel dans l'organisme humain, ledit dispositif comprenant (1) un moyen non invasif mesurant les paramètres vasculaires caractérisant l'état du Système Nerveux Végétatif (SNV), (2) moyens d'analyse pour calculer les valeurs des Facteurs de Contrainte du Système Nerveux Végétatif (FCSNV) à partir des valeurs mesurées des paramètres vasculaires, **caractérisé en ce que** le dispositif comprend (3) des moyens d'analyse pour calculer les FCSNV et/ou lesdites bandes de valeurs de paramètres correspondant à différentes séries de mesures ayant des valeurs de coupure inhérentes qui limitent les bandes, (4) moyens d'analyse pour comparer les FCSNV du patient et/ou lesdites valeurs de paramètres par rapport à celles des bandes (3) ci-dessus, par rapport à la coupure limitant les FCSNV et/ou lesdites valeurs de paramètres de ces bandes et par rapport aux FCSNV du patient et/ou lesdites valeurs de paramètres déterminées plus tôt, et (5) moyens d'analyse pour le diagnostic du patient comme correspondant au moins potentiellement à au moins l'une desdites séries de mesures ou comme ayant une contrainte du SNV croissante ou décroissante dans le temps.

**2.** Le dispositif selon la revendication 1 incluant en outre d'autres moyens d'analyse calculant les probabilités de FCSNV du patient et/ou lesdites valeurs de paramètres à tomber dans lesdites bandes.

**3.** Le dispositif selon la revendication 1 incluant en outre des moyens d'analyse calculant les probabilités véridiques des FCSNV du patient et/ou lesdites valeurs de paramètres pour correspondre à au moins l'une desdites bandes.

**4.** Le dispositif selon la revendication 1 dans lequel ledit moyen non invasif mesure au moins l'un des groupes de paramètres vasculaires / cardio-vasculaires parmi les suivants : conductance vasculaire totale, élasticité vasculaire, index d'élasticité de la grande artère, index d'élasticité de la petite artère, conductance vasculaire du système de fréquence du pouls, ou une combinaison de cela.

**5.** Le dispositif selon la revendication 1 dans lequel ledit moyen non invasif mesure les crêtes et creux du Volume Sanguin (VS) dans au moins une période de pleine apoplexie cardiaque, au moins les pics du VS dus au ventricule gauche du coeur et aorte et les creux correspondants à la fermeture de la valvule aortique et/ou du VS par représentation oscillographique.

**6.** Le dispositif selon la revendication 1 dans lequel ledit moyen non invasif mesure les crêtes et creux du VS/dt (vitesse de variation) dans au moins une période de pleine apoplexie cardiaque, au moins les pics du VS dus au ventricule gauche du coeur et aorte et les creux correspondants à la fermeture de valvule aortique et/ou du VS/dt par représentation oscillographique.

**7.** Le dispositif selon la revendication 1 dans lequel ledit moyen non invasif mesure les crêtes et creux de la Tension Artérielle (TA) dans au moins une période de pleine apoplexie cardiaque, au moins les pics de la TA dus au ventricule gauche du coeur et aorte et les creux correspondants à la fermeture de la valvule aortique et/ou de la TA par représentation oscillographique.

**8.** Le dispositif selon la revendication 1 dans lequel ledit moyen non invasif mesure les crêtes et creux de la TA/dt dans au moins une période de pleine d'apoplexie cardiaque, au moins les pics de la TA/dt dus au ventricule gauche du coeur et aorte et les creux correspondants à la fermeture de la valvule aortique et/ou de la TA/dt par représentation oscillographique.

**9.** Le dispositif selon la revendication 1 dans lequel ledit moyen non invasif mesure les crêtes et creux du VS/dt2 (accélération de variation) dans au moins une période complète d'apoplexie cardiaque, au moins les pics de VS/dt2 dus au ventricule gauche du coeur et aorte et les creux correspondants à la fermeture de la valvule aortique et/ou du VS/dt2 par représentation oscillographique.

10. Le dispositif selon la revendication 1 dans lequel ledit moyen non invasif mesure les crêtes et creux de la TA/dt2 dans au moins une période complète d'apoplexie cardiaque, au moins les pics de la TA/dt2 dus au ventricule gauche du coeur et aorte et les creux correspondants à la fermeture de la valvule aortique et/ou de la TA/dt2 par représentation oscillographique.

11. Le dispositif selon les revendications 5 à 10 dans lequel ledit moyen non invasif est sélectionné parmi le groupe suivant : une sonde photopléthysmographe, un appareil mesurant la pression physique, une sonde rhéographique, une sonde ultrasonique, et une sonde à rayon X.

12. Le dispositif selon les revendications 5 et 11 incluant en outre d'autres moyens de calcul transformant le VS par rapport aux réponses temporelles en VS/dt par rapport aux réponses temporelles par différenciation.

13. Le dispositif selon les revendications 6 et 11 incluant en outre d'autres moyens de calcul transformant le VS/dt par rapport aux réponses temporelles en VS par rapport aux réponses temporelles par intégration.

14. Le dispositif selon les revendications 5 et 11 incluant en outre d'autres moyens de calcul transformant le VS par rapport aux réponses temporelles en VS/dt2 par rapport aux réponses temporelles par double différenciation.

15. Le dispositif selon les revendications 6 et 11 incluant en outre d'autres moyens de calcul transformant le VS/dt par rapport aux réponses temporelles en VS/dt2 par rapport aux réponses temporelles par différenciation.

16. Le dispositif selon les revendications 9 et 11 incluant en outre d'autres moyens de calcul transformant le VS/d2 par rapport aux réponses temporelles en VS/dt par rapport aux réponses temporelles par intégration.

17. Le dispositif selon les revendications 9 et 11 incluant en outre d'autres moyens de calcul transformant le VS/d2 par rapport aux réponses temporelles en VS par rapport aux réponses temporelles par double intégration.

18. Le dispositif selon les revendications 7 et 11 incluant en outre d'autres moyens de calcul transformant la TA par rapport aux réponses temporelles en TA/dt par rapport aux réponses temporelles par différenciation.

19. Le dispositif selon les revendications 8 et 11 incluant en outre d'autres moyens de calcul transformant la TA/dt par rapport aux réponses temporelles en TA par rapport aux réponses temporelles par intégration.

20. Le dispositif selon les revendications 7 et 11 incluant en outre d'autres moyens de calcul transformant la TA par rapport aux réponses temporelles en TA/dt2 par rapport aux réponses temporelles par double différenciation.

21. Le dispositif selon les revendications 8 et 11 incluant en outre d'autres moyens de calcul transformant la TA/dt par rapport aux réponses temporelles en TA/dt2 par rapport aux rapport aux réponses temporelles par différenciation.

22. Le dispositif selon les revendications 10 et 11 incluant en outre d'autres moyens de calcul transformant la TA/dt2 par rapport aux réponses temporelles en TA/dt par rapport aux réponses temporelles par intégration.

23. Le dispositif selon les revendications 10 et 11 incluant en outre d'autres moyens de calcul transformant la TA/dt2 par rapport aux réponses temporelles en TA par rapport aux réponses temporelles par double intégration.

24. Le dispositif selon les revendications 5, 13 et 17 dans lequel lesdits moyens d'analyse pour calculer les valeurs des FCSNV à partir des valeurs de paramètres vasculaires mesurées calcule cette valeur selon l'expression suivante:

$$I = (A-C)/(B-C),$$

où I est FCSNV,
A est le VS correspondant à la poussée d'aorte,
B est le VS correspondant à la poussée du ventricule gauche du coeur,
C est le VS correspondant à la fermeture de la valvule aortique.

25. Le dispositif selon la revendication 24 dans lequel A et B sont les valeurs maximales et C est la valeur minimale du VS correspondant.

26. Le dispositif des revendications 7, 19 et 23 dans lequel lesdits moyens d'analyse pour calculer la valeur des FCSNV à partir des valeurs de paramètres vasculaires (cardio-vasculaires) mesurés calcule cette valeur selon l'expression suivante :

$$I = (A-C)/(B-C),$$

où I est FCSNV,
A est la TA correspondant à la poussée d'aorte,
B est la TA correspondant à la poussée du ventricule gauche du coeur,
C est la TA correspondant à la fermeture de la valvule aortique.

27. Le dispositif selon la revendication 26 dans lequel A et B sont les valeurs maximales et C est la valeur minimale de la TA correspondante.

28. Le dispositif selon les revendications 6, 12 et 16 dans lequel lesdits moyens d'analyse pour calculer la valeur des FCSNV à partir des valeurs de paramètres vasculaires mesurées calcule cette valeur selon l'expression suivante :

$$I = (A-C)/(B-C),$$

où I est FCSNV,
A est le VS/dt correspondant à la poussée d'aorte,
B est le VS/dt correspondant à la poussée du ventricule gauche du coeur,
C est le VS/dt correspondant à la fermeture de la valvule aortique.

29. Le dispositif selon la revendication 28 où A et B sont les valeurs maximales et C est la valeur minimale du VS/dt correspondant.

30. Le dispositif selon les revendications 8, 18 et 22 dans lequel lesdits moyens d'analyses pour le calcul de la valeur de FCSNV à partir des valeurs paramètres vasculaires mesurés calcule cette valeur selon l'expression suivante:

$$I = (A-C)/(B-C),$$

où I est FCSNV,
A est la TA/dt correspondant à la poussée d'aorte,
B est la TA/dt correspondant à la poussée du ventricule gauche du coeur,
C est la TA/dt correspondant à la fermeture de la valvule aortique.

31. Le dispositif selon la revendication 30 dans lequel A et B sont les valeurs maximales et 30 C est la valeur minimale de la TA/dt correspondante.

32. Le dispositif selon les revendications 9, 14 et 15 où lesdits moyens d'analyse pour calculer les valeurs de FCSNV à partir des valeurs mesurées des paramètres vasculaires calcule cette valeur selon l'ex-

pression suivante:

$$I = (A-C)/(B-C),$$

où I est FCSNV,
A est le VS/dt2 correspondant à la poussée aortique,
B est le VS/dt2 correspondant à la poussée du ventricule gauche du coeur,
C est le VS/dt2 correspondant à la fermeture de la valvule aortique.

33. Le dispositif selon la revendication 32 dans lequel A et B sont les valeurs maximales et C est la valeur minimale du VS/dt2 correspondant.

34. Le dispositif selon les revendications 10, 20 et 21 dans lequel lesdits moyens d'analyse pour calculer les valeurs de FCSNV à partir des valeurs mesurées des paramètres vasculaires calcule cette valeur selon l'expression suivante:

$$I = (A-C)/(B-C),$$

où I est FCSNV,
A est la TA/dt2 correspondant à la poussée aortique,
B est la TA/dt2 correspondant à la poussée du ventricule gauche du coeur,
C est la TA/dt2 correspondant à la fermeture de la valvule aortique.

35. Le dispositif selon la revendication 34 dans lequel A et B sont les valeurs maximales et C est la valeur minimale de la TA/dt2 correspondante.

36. Le dispositif selon la revendication 1 dans lequel lesdits moyens d'analyse pour diagnostiquer le patient, le diagnostic FCSNV excédant 0,3, étant mesuré selon les valeurs maximales A et B, correspondant à l'état de contrainte du SNV du à l'abus de drogues et substances toxiques, alcoolisme, intoxications à médicaments et à d'autres substances et désordre hormonal du fait d'une maladie ou de processus naturels inhérents à l'organisme humain.

37. Le dispositif selon la revendication 11 dans lequel ledit moyen non invasif est connecté directement ou par d'autres moyens à un oscilloscope et/ou directement ou par d'autres moyens à un ordinateur.

38. Le dispositif selon la revendication 11 comprenant en outre un élément convertisseur pour la conversion du signal analogique vers le digital à partir dudit moyen non invasif.

**39.** Le dispositif selon la revendication 38 dans lequel ledit élément convertisseur est incorporé audit moyen non invasif.

**40.** Le dispositif selon la revendication 38 dans lequel ledit élément convertisseur est un ordinateur.

**41.** Le dispositif selon la revendication 11 comprenant en outre un élément d'amplification.

**42.** Le dispositif selon la revendication 41 dans lequel ledit élément d'amplification est incorporé audit moyen non invasif.

**43.** Le dispositif selon les revendications 11 et 37 comprenant en outre un élément de pontage pour bloquer le composant constant dudit signal du moyen non invasif à partir dudit oscilloscope et/ou ordinateur.

**44.** Le dispositif selon les revendications 38 à 42 comprenant en outre une fourniture d'alimentation électrique de tension en courant continu pour ledit élément convertisseur et/ou pour ledit élément d'amplification, ladite fourniture de tension en courant continu étant sélectionnée parmi le groupe suivant : un oscilloscope ou un ordinateur.

**45.** Le dispositif selon les revendications 12 à 23 dans lequel ledit élément de calcul est incorporé audit moyen non invasif.

**46.** Le dispositif des revendications 12 à 23 dans lequel ledit élément de calcul est un ordinateur.

**47.** Le dispositif selon la revendication 1 dans lequel un ordinateur est utilisé comme moyen d'analyse pour calculer les FCSNV et/ou lesdites bandes de valeurs de paramètres et/ou pour comparer les FCSNV du patient et/ou lesdites valeurs de paramètres et/ou pour diagnostiquer le patient.

Fig.2. BP/dt vs time responses typical for persons with strained VNS

Fig. 1. BP/dt vs.time response typical for persons with unsrained VNS

Fig.4. VNSSF value probabilities to correspond to drug-free (dashed curve) and to drug abuse groups (solid curve)

Fig.3. VNSSF distribution for drug-free (gray) and drug abuse groups (dark)

Fig.5. VNSSF integral probabilities for drug-free (dashed curve) and drug abuse (solid curve)

**Fig.6 Device according to the present invention**

1 is Non-invasive means measuring vascular (cardiovascular) parameters
2 is Amplifying means
3 is Analog-to-digital converting means
4 is Bypassing means
5 is Analysis means for calculating VNSSF value
6 is Analysis means for calculating parameters values bands
7 is Analysis means for comparing of the patients VNSSF values against that in the bands
8 is Analysis means for calculating VNSSF probabilities of patient's VNSSF to fall within bands calculated by (6)
9 is Analysis means calculating true probabilities of patient's VNSSF values to correspond to at least one of the said bands.
10 is Analysis means for comparing of the patients VNSSF values against the values determined for him earlier
11 is DC voltage supply means
12 is Diagnosinng means
13 is Computer

EP 1 351 605 B1

**Fig.7 Device according to preferred embodiment of the present invention**

1 is Non-invasive means measuring vascular (cardiovascular) parameters Incorporating (2) and (4)
2 is Amplifying means
3 is Analog-to-digital converting means
4 is Bypassing means
5 is Analysis means for calculating VNSSF value
6 is Analysis means for calculating parameters values bands
7 is Analysis means for comparing of the patients VNSSF values against that in the bands
8 is Analysis means for calculating VNSSF probabilities of patient's VNSSF to fall within bands calculated by (6)
9 is Analysis means calculating true probabilities of patient's VNSSF values to correspond to at least one of the said bands.
10 is Analysis means for comparing of the patients VNSSF values against the values determined for him earlier
11 is DC voltage supply means
12 Is Diagnosinng means
13 is Computer incorporating (3) and (5) through (12)
14 is USB or Com computer port being DC voltage supply means

EP 1 351 605 B1